Europäisches Patentamt

European Patent Office

Office européen des brevets

(19)

(11) Veröffentlichungsnummer : **0 513 400 B1**

(12) # EUROPÄISCHE PATENTSCHRIFT

(45) Veröffentlichungstag der Patentschrift :
17.03.93 Patentblatt 93/11

(51) Int. Cl.$^5$ : **A61K 31/60**

(21) Anmeldenummer : **91106203.2**

(22) Anmeldetag : **18.04.91**

(54) **Verwendung von 2-Ethoxybenzoesäure zur Herstellung eines Arzneimittels als Analgetikum, Antipyretikum und/oder Antiphlogistikum.**

(43) Veröffentlichungstag der Anmeldung :
19.11.92 Patentblatt 92/47

(45) Bekanntmachung des Hinweises auf die
Patenterteilung :
17.03.93 Patentblatt 93/11

(84) Benannte Vertragsstaaten :
AT BE CH DE ES FR GB GR IT LI NL SE

(56) Entgegenhaltungen :
GB-A- 1 049 036
JOURNAL OF PHARMACOBIO-DYNAMICS,
Band 4, Nr. 1, Januar 1981; T.KIMURA et al.,
Seiten 35-41.
THE JOURNAL OF PHARMACOLOGY AND EX-
PERIMENTAL THERAPEUTICS, Band 136, Nr.
2, Mai 1962, US; C.DAVISON et al., Seiten
226-231.
BIOCHEMICAL PHARMACOLOGY, Band 36,
Nr. 7, 01 April 1987, GB; J.NAKAMURA et al.,
Seiten 1171-1174.
INDIAN JOURNAL OF EXPERIMENTAL BIO-
LOGY, Band 16, Nr. 11, November 1978;
S.R.NAIK et al., Seiten 1169-1174.
PHARMAZEUTISCHE STOFFLISTE, 7. Auflage,
1990; Seiten 314-315.

(73) Patentinhaber : **DR. R. PFLEGER CHEMISCHE
FABRIK GMBH
Emil-Kemmer-Strasse 33
W-8605 Hallstadt (DE)**

(72) Erfinder : **Bertholdt, Heinz, Dr.
Birkenstrasse 3
W-8608 Memmelsdorf (DE)**
Erfinder : **Michalczyk, Dieter
Baumfeldstrasse 17
W-8608 Drosendorf/DE (DE)**
Erfinder : **Lieb, Herbert, Dr.
Krötleinstrasse 18
W-8600 Bamberg (DE)**
Erfinder : **Härtl, Rudolf, Dr.
Unterer Kapellberg 56
W-8605 Hallstadt (DE)**

(74) Vertreter : **Fleck, Thomas, Dr.Dipl.-Chem.
Patentanwälte Raffay, Fleck & Partner
Postfach 32 32 17
W-2000 Hamburg 13 (DE)**

EP 0 513 400 B1

## Beschreibung

Seit mehreren Jahrzehnten ist die analgetische, antipyretische und antiphlogistische Wirkung von Ethenzamid (2-Etoxybenzamid) bekannt (vgl. hierzu beispielsweise die GB-PS 656 746). Ethenzamid ist bekanntermaßen das Amid des Salicylsäureethyläthers.Als dessen Derivat gehört es zur Gruppe der nichtsteroidalen Antirheumatika (NSAR). Hinsichtlich seiner analgetischen Potenz soll es der Acetylsalicylsäure (ASS) mindestens ebenbürtig sein. Es wird häufig als Mittel der Wahl angesehen, wenn andere Analgetika, wie z.B. ASS, Unverträglichkeitsreaktionen hervorrufen oder bestehende Blutungsneigungen verstärken. So sind seit langem die Schwierigkeiten bekannt, die Patienten bei der Einnahme von ASS-Tabletten haben (vgl. DE-PS'en 963 270 und 30 00 743). Die Arzneimittelforschung war deshalb schon immer daran interessiert, weitere Alternativen für ASS zu finden.

Ebenfalls sind verschiedene Herstellungsverfahren für Ethenzamid bekannt (vgl. hierzu Beilstein's Handbuch der organischen Chemie, 4. Aufl. , Springer-Verlag, 1983, E IVC, Seite 175.

Die Markteinführung von Ethenzamid erfolgte z.B. in Deutschland 1925, in Frankreich 1942 und in Japan 1954. Ethenzamid wird in mehr als 15 Ländern weltweit verwendet. Der Weltverbrauch lag 1985 bis 1989 zwischen 220 und 250 t/Jahr ohne China und Ost-Block. Das entspricht bei 0,25 g/Dosis einem Verbrauch von etwa 1,0 Milliarden-Dosiseinheiten/Jahr. Toxikologie und Pharmakologie von Ethenzamid sind gut belegt, während de Dokumentation der klinischen Wirksamkeit auf wenigen älteren Studien beruht, die meist mit Kombinationspräparaten durchgeführt worden sind.

Beim Ethenzamid sind im Vergleich zur Salicylsäure die beiden reaktiven Gruppen (phenolische OH-Gruppe und saure COOH-Gruppe) blockiert. Dadurch wird die chemische und physiologische Reaktivität der Substanz im Vergleich zur Salicylsäure verändert. Es wurde dadurch auch zu einer idealen Komponente für analgetische Kombinationspräparate.

Wie bei vielen altbekannten und bewährten Pharmaka sind auch bei Ethenzamid praktisch keine fundierte Erkenntnisse zur Pharmakokinetik und zum Metabolismus beim Menschen vorhanden.

Wegen seiner stark begrenzten Löslichkeit in Wasser wird Ethenzamid oral in Tablettenform bzw. rektal als Suppositorium verabeicht; eine parenterale Applikation in Form einer Injektionslösung fehlt ebenso wie eine Lösung zur oralen Anwendung, die eine rasch einsetzende Wirkung gewährleisten.

Der Erfindung liegt deshalb die Aufgabe zugrunde, ein alternatives Arzneimittel zur Verfügung zu stellen, um die geschilderten Nachteile zu vermeiden.

Diese Aufgabe wird durch die Verwendung von 2-Ethoxybenzoesäure oder deren Salzen zur Herstellung einer Arzneizubereitung als Analgetikum, Antipyretikum und/oder Antiphlogistikum gelöst.

Erfindungsgemäß wird erstmalig gezeigt, daß das analgetisch wirksame Ethenzamid lediglich Pro-Drug-Funktion hat; das eigentliche Wirkprinzip ist sein Metabolit, nämlich die 2-Ethoxybenzoesäure, deren analgetische Potenz bislang nicht erkannt worden ist und nunmehr unmittelbar therapeutisch vorteilhaft genutzt werden kann.

Dem Fachmann ist natürlich geläufig, daß sich Säureamide ähnlich wie Ester mit Wasser in Gegenwart von Alkalien oder Säuren verseifen lassen. Trotz dieser jahrzehntelang bekannten Tatsache finden sich weder in der Literatur noch in der Praxis irgendwelche Hinweise auf die medizinische Anwendung der 2-Ethoxybensoesäure.

Vielmehr führen veröffentlichte Untersuchungsergebnisse in die Leere, da sie der Ethoxybenzoesäure keine klinische Bedeutung zumessen (vgl. J. Pharmacol.Exptl. Therap. 136, Seite 230, re. Spalte (1962), sowie J.-P. Dupin et al in Il Farmaco, 41 (31), 1986, Tab. I (Produkt VI) sowie P. Formijne in Koninkl. Med. Akad. Wetenschap, Proc., Ser.C 58, 1955, Seite 576).

Überraschenderweise haben pharmakokinetische Untersuchungen erkennen lassen, daß Ethenzamid nach Resorption vollständig und ausschließlich zur 2-Ethoxybenzoesäure metabolisiert wird, deren Cmax bei peroraler Applikation schon nach 2 Stunden erreicht wird.

Durch pharmakodynamische Untersuchungen wurde erstmalig nachgewiesen, daß 2-Ethoxybenzoesäure analgetisch wirksam ist und als Arzneimittel genutzt werden kann.

Erfindungsgemäß wurde gezeigt, daß auch nach rektaler Anwendung Ethenzamid in gleicher Weise zur 2-Ethoxybenzoesäure metabolisiert wird. Die maximalen Blutspiegel werden hier erwartungsgemäß später, nach ca. 6 Stunden, erreicht. Nach Abschluß der Resorptionsphase ist der weitere Verlauf der Serumkonzentrationskurven jedoch deckungsgleich.

Die Erfindung zeigt also, daß die pharmakodynamischen Eigenschaften von Ethenzamid in Wirklichkeit auf seinem Metaboliten, der 2-Ethoxybenzoesäure, beruhen, der zum Unterschied des schwer wasserlöslichen Ethenzamids als Salz eine gute Wasserlöslichkeit aufweist und sich deshalb vorteilhafterweise auch für die Herstellung von Tropf- oder Injektionslösungen einsetzen läßt.

Weitere Vorteile und Merkmale gehen aus den Unteransprüchen hervor, die auch gemeinsam mit dem Hauptanspruch von erfinderischer Bedeutung sein können.

Im folgenden wird anhand der Abbildungen die Erfindung zum besseren Verständnis anhand der Bioverfügbarkeitsstudien erläutert.

Es zeigt;

Abb. 1 den 2-Ethoxybenzoesäure-Plasmaspiegel im 24-stündigen Verlauf nach oraler Applikation im Vergleich zum Ethenzamid nach oraler bzw. rektaler Applikation;

Abb. 2 die NI-Amplitude des Laser-SEPs für Ethenzamid, ASS bzw. Placebogabe; und

Abb. 3 einer Darstellung der P2-Amplitude des Laser-SEPs für Ethenzamid, ASS bzw. Placebogabe.

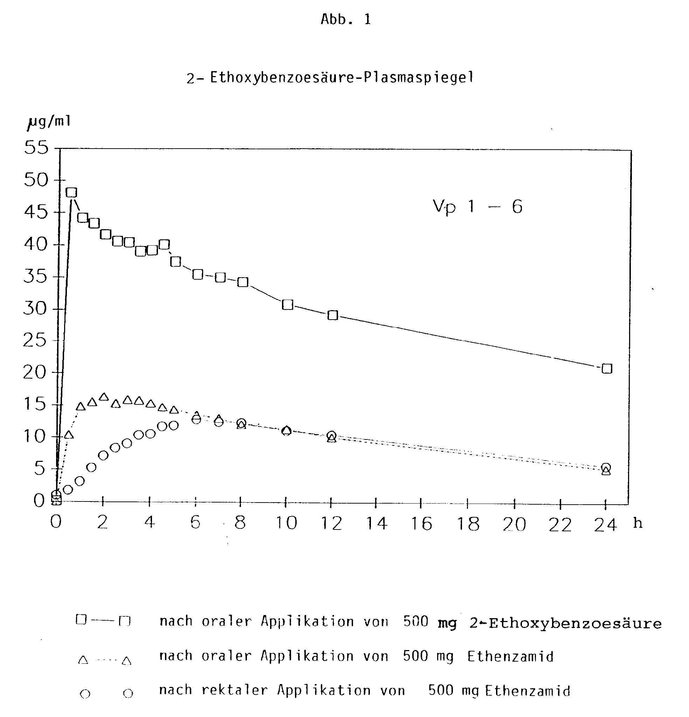

Nach oraler Gabe einer Lösung von 2-Ethoxybenzoesäure (500 mg) sind die Plasmaspiegel über den gesamten Untersuchungszeitraum um den Faktor 3 höher als die entsprechenden Spiergel nach oraler oder rektaler Gabe der Ethenzamid-Präparationen (Tabletten und Suppositorien zu je 500 mg Ethenzamid). Die Resorption erfolgt rasch. Bereits nach 30 Minuten sind die Maxima erreicht. Die Serumkonzentration verläuft aber parallel zu der nach oraler bsw. rektaler Applikation von Ethenzamid. Durch die schnellere Resorption der freien Säure und ihre 3-fach höhere Bioverfügbarkeit im Vergleich zu der aus Ethenzamid entstehenden 2-Ethoxybenzoesäure kann die Dosis bei gleicher Wirksamkeit erheblich reduziert werden. Da Salicylamid im Serum nicht nachgewiesen werden konnte, ist damit die bisherige Auffassung widerlegt, daß Salicylamid der Hauptmetabolit von Ethenzamid ist (s. Abb. 1).

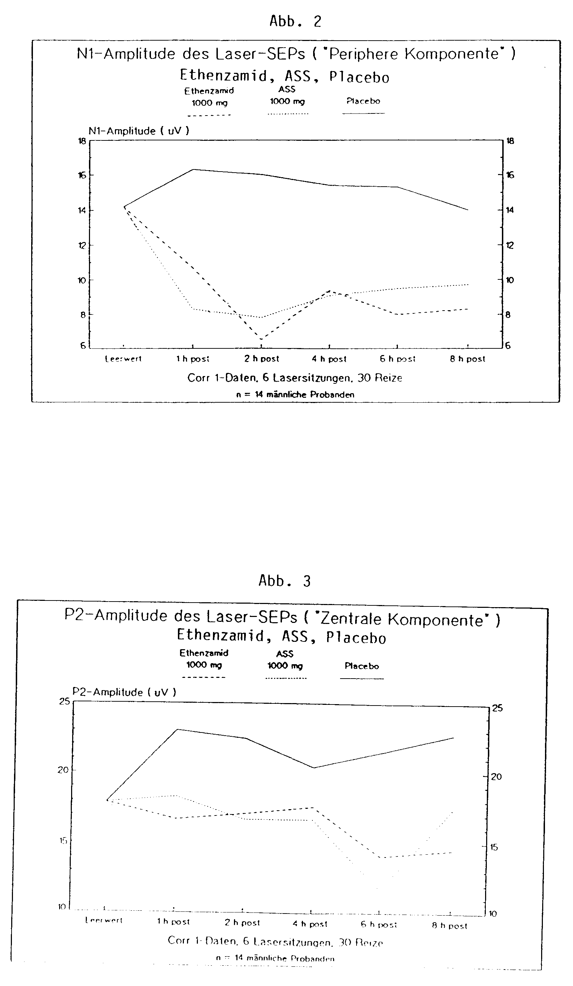

Gleichzeitig mit den pharmakokinetischen Untersuchungen wurde die analgetische Potenz von Ethenzamid im Vergleich zu ASS bestimmt. Laser evozierte somatosensorische Potentiale unter Vigilanzkontrolle wurden als Nachweis der Analgesie verwendet. Mit dieser Methode ließen sich auch periphere (N-1) und zentrale (P2) analgetische Wirkungen nachweisen, die gegenüber Placebo auf dem 0,1 % Niveau statistisch signifikant und mit denen von ASS vergleichbar waren. Wirkmaxima wurden nach 1 bis 2 Stunden erreicht und hielten bis zum Ende der Meßperiode (8 Stunden) an. Die analgetische Wirkung korreliert mit den Serumspiegeln der 2-Ethoxybenzoesäure. Auch bei der Messung der zentralen Komponente fiel der lange anhaltende Zeitgang der Wirkung auf ( > 8 h) (s. Abb. 2 und 3).

Durch weitere Untersuchungen konnte nachgewiesen werden, daß Ethenzamid als Pro-Drug bzw. 2-Ethoxybenzoesäure im Gegensatz zu Acetylsalicylsäure keine hemmende Wirkung auf die Thrombozytenaggregation hat.

Nachfolgend werden Rezepturen für erfindungsgemäße Arzneistoffzubereitungen aufgeführt, die den Wirkstoff 2-Ethoxybenzoesäure enthalten. Es dürfte jedoch für den Fachmann einleuchten, daß Abwandlungen dieser Rezeptur in üblichen Bereichen möglich sind, ohne daß der Rahmen der vorliegenden Erfindung verlassen wird:

Die Herstellung wässriger Lösungen in Sirupform bzw. als Tropflösung erfolgt unter Verwendung von Salzen der 2-Ethoxybenzoesäure (EBS), von Süßungsmitteln (z. B. Sorbitlösung, Glycerin, Zuckersirup, Maltitose-Sirup, Saccharin/Cyclamat), Konservierungsmitteln (z. B. Parabene, Äthanol), Geschmacks-Korrigentien (z.B. Himbeer-, Banane-, Malzaroma) und Wasser.

Beispiel 1 : Darreichungsform: Sirup (250 mg EBS/5 ml)

```
 5,66 g   EBS, Natriumsalz
50,00 g   Zuckersirup
 0,18 g   p-Hydroxybenzoesäuremethylester
 0,02 g   p-Hydroxybenzoesäurepropylester
 2,00 g   Himbeeraroma, künstlich
42,14 g   Wasser
```

Beispiel 2 : Darreichungsform: Tropflösung (250 mg EBS/2 ml)

```
28,31 g   EBS, Natriumsalz
 5,00 g   Glycerin
20,00 g   Ethanol
 2,00 g   Himbeeraroma, künstlich
44,69 g   Wasser
```

Die Herstellung von Injektionslösungen erfolgt unter Verwendung von salzen der 2-Ethoxybenzoesäure und Wasser; die erhaltene Lösung wird unter Verwendung von verdünnter Salzsäure auf pH 6,8 bis 7,2 eingestellt, partikelfrei filtriert, ampulliert und sterilisiert.

Beispiel 3: Darreichungsform: Injektionslösung (500 mg/5ml

```
     11,32 g   EBS, Natriumsalz

            verdünnte Salzsäure bis pH 6,8 - 7,2

   ad 100,0  ml Wasser für Injektionszwecke.
```

Die Herstellung von festen Zubereitungen wie Kapseln und Tabletten erfolgt in an sich bekannter Weise, indem ein Salz der EBS mit einem Füllstoff (z. B. Stärke, Milchzucker, Cellulose, Dicalciumphosphat) versetzt, mit einem geeigneten Granulierhilfsmittel (z. B. Gelatine, Polyvinylpyrrolidon), lösliche Stärke, lösliche Cellulosederivate) in an sich bekannter Weise granuliert und das Granulat entweder in Hartgelatinekapseln eingebracht oder nach Zusatz eines Gleitmittels (z.B. höhere Fettsäuren und deren Erdalkalisalze, Polyglykol, Paraffin) in an sich bekannter Weise tablettiert wird:

Beispiel 4 : Darreichungsform: Kapsel (250 mg EBS/Kapsel)

```
283,07 g   EBS, Natriumsalz
120,00 g   Maisstärke
 40,00 g   Milchzucker

           werden gemischt, mit einer wäßrigen Lösung von

 12,00 g   löslicher Stärke

           granuliert;

           dem getrockneten Granulat werden

  2,00 g   Magnesiumstearat

           zugemischt (= Kapselfüllgut)

  457 mg   Kapselfüllgut, entsprechend 250 mg EBS, sind pro
           Einzelkapsel zu dosieren.
```

Beispiel 5 : Darreichungsform: Tabletten (250 mg EBS/Tabl.)

```
283,07 g   EBS, Natriumsalz
150,00 g   Maisstärke
 89,93 g   Milchzucker
 80,00 g   Cellulosepulver

           werden gemischt, mit einer Lösung von

 18,00 g   Kollidon 25 in 40 ml Wasser granuliert.

           Das getrocknete Granulat wird nach Zusatz von

 25,00 g   PVP, cross linked
  4,00 g   Magnesiumstearat

           gemischt und zu Tabletten mit einem Tablettengewicht
           von 650 mg verpreßt.
```

4

Die Herstellung von Suppositorien erfolgt in an sich bekannter Weise unter Verwendung von EBS und Hartfett.

Beispiel 6 : Darreichungsform: Suppositorien (250 mg EBS/Supp.)

```
12,5    g  EBS

        werden in der 40-45° warmen Schmelze von

87,5    g  Hartfett

        gelöst und in an sich bekannter Weise in 2 g-
        Suppositorienformen eingebracht.
```

## Patentansprüche

1.  Verwendung von 2-Ethoxybenzoesäure oder deren Salzen zur Herstellung einer Arzneizubereitung als Analgetikum, Antipyretikum und/oder Antiphlogistikum.

## Claims

1.  Use of 2-ethoxybenzoic acid or its salts for producing a medicament formulation as an analgesic, antipyretic and/or antiphlogistic.

## Revendications

1.  Utilisation de l'acide 2-éthoxy-benzoique ou leur sels pour l'obtention d'un medicament préparation destiné comme analgésique, antipyrétique et/ou antiinflammatoire.

Abb. 1

2- Ethoxybenzoesäure-Plasmaspiegel

□——□ nach oraler Applikation von 500 **mg** 2-Ethoxybenzoesäure

△ ···· △ nach oraler Applikation von 500 mg Ethenzamid

○ ○ nach rektaler Applikation von 500 mg Ethenzamid

Abb. 2

N1-Amplitude des Laser-SEPs ( "Periphere Komponente" )
Ethenzamid, ASS, Placebo

Abb. 3

P2-Amplitude des Laser-SEPs ( "Zentrale Komponente" )
Ethenzamid, ASS, Placebo

7